# EUROPEAN PATENT APPLICATION

(11) **EP 2 380 482 A1**
(43) Date of publication of application: **26.10.2011**
(21) Application number: 10160529.3
(22) Date of filing: 21.04.2010
(51) Int. Cl.: A61B 1/00, A61B 1/06, A61B 1/32, A61B 5/00

(54) **Extending image information**

(71) Applicant: Koninklijke Philips Electronics N.V., 5621 BA Eindhoven (NL)
(72) Inventor: The designation of the inventor has not yet been filed
(74) Representative: Damen, Daniel Martijn

(57) **Abstract**

The present invention relates to a system for extending microscopy information, where the microscopy information is image information from a first region 118 of an associated sample, the first region being imaged with an imaging system. The extension of the microscopy information originates from probing a larger second region 116 by photons which are emitted at an exit position 128 and collected at en entry position 130. The exit position and the entry position are spatially separated so that so that average spectral information of photons emitted from the exit position and collected at the entry position, is dependent on the second region 116 of the associated sample, the second region 116 being larger than the first region 118.

## Description

### FIELD OF THE INVENTION

The present invention relates to the field of extending image information, in particular, the present invention relates to extending image information with average spectral information.

### BACKGROUND OF THE INVENTION

The interpretation of a microscope image of a sample may depend on parameters of the sample. For instance, when imaging a part of a sample, such as a tissue or foodstuffs, the sample may have a lipid-water ratio which may be beneficial information for correct interpretation of the image. In oncology it is important to identify the abnormal tissue from normal tissue as well as grade the abnormal tissue. One way to obtain such information is to take a biopsy and send this to the pathology department for diagnosing the tissue. A problem here is that most of these biopsies are taken blindly without feedback of what tissue is in front of the biopsy device. Hence there is a risk that the biopsy is taken at the wrong place.

Furthermore, sending a sample of tissue to the pathology department and asking for a frozen section takes typically at least half an hour. Hence it is not standard practice to do this in all operations. It would certainly improve the situation if it were possible to examine the tissue in a more practical, simple, cheaper, and faster way. A way to improve this situation is by bringing microscopic tissue inspection into a needle-like device to allow tissue inspection in front of the needle. The reference WO 2007/123518 A1 describes an apparatus which enables an operator to simultaneously collect images and spectroscopic information from a region of interest using a multiple modality imaging and/or spectroscopic probe. However, the added information may still be less than optimal for interpreting the images. Since no staining can be used during these microscopic inspections the image contrast is in general of less quality than what can be achieved in the pathology department. A problem is then how to interpret the information contained in the microscopic imaging, and it would be advantageous to extend the information, so that interpretation would be facilitated.

Hence, an improved apparatus for extending image information would be advantageous, and in particular an improved, more efficient, cheap, simple, fast and/or reliable apparatus for extending image information would be advantageous.

### SUMMARY OF THE INVENTION

In particular, it may be seen as an object of the present invention to provide a system that solves the above mentioned problems of the prior art concerning how to interpret the information contained in the microscopic imaging.

It is a further object of the present invention to provide an alternative to the prior art.

Thus, the above described object and several other objects are intended to be obtained in a first aspect of the invention by providing a system for obtaining extended microscopy information, comprising
- a first light source for imaging,
- a spectrometer comprising
- a second light source, and
- an optical detector, and
- an interventional device, where the interventional device has
- imaging optics capable of guiding light from the first light source so to perform imaging of a first region of an associated sample,
- a first guide for guiding photons from the second light source to an exit position on a distal end of the interventional device, the photons being emittable from the exit position, and
- a second guide for guiding photons from an entry position on the distal end of the interventional device and to the optical detector,
wherein the exit position and the entry position are spatially separated and spatially oriented so that, upon positioning the distal end of the interventional device adjacent to the associated sample, an average spectral information is obtainable from photons collectable at the entry position, the average spectral information comprises information about a second region of the associated sample, and
wherein the exit position and the entry position are arranged so that the second region is larger than the first region.

The invention is particularly, but not exclusively, advantageous for extending the information of microscopic inspections having image contrast which is in general of less quality than what can be achieved in the pathology department. A possible advantage of extending the information is that the improved interpretation of the images is facilitated. By providing the exit position as described so that the second region is larger than the first region, the microscopic information - stemming from the first region, such as the field of view of the imaging optics - may be extended with additional global information on the biological composition of the tissue - where the global information stems from the larger second region. The first and second guide together with the spectrometer provide averaged information on certain tissue constituents such as blood content, blood oxygenation, water and fat content. Thus, and advantage of having the second region larger than the first region, is that a larger region is averaged, so that the average spectral information from the spectrometer and the first and second guide, is less susceptible to relatively small regions where certain small constituents could otherwise dominate the spectral information. In some embodiments, the first or second guide may be identical with a guide in the imaging system. In particular, the imaging optics may comprise an imaging guide which may serve as either first or second guide.

The first region may be a volume defined as the imaged region, such as the field of view of the imaging optics. The second region may be a volume traversed by the diffusive photons emitted from the exit position and collected at the entry position. The first and second region may be overlapping or non-overlapping. In particular, the first region may be comprised within the second region.

The first and second guide and the imaging guide are understood to be light guides, such as optical fibres.

A spectrometer is understood as is common in the art. It is understood, that the spectrometer comprises means for selecting wavelengths, such as transmission filters or gratings. Alternatively, wavelength specific light sources, such as light emitting diodes or LASERs, may be used or wavelength specific optical detectors may be used. A spectral filtration may occur at different places in the system, for instance it may occur between the second light source and the interventional device, it may occur in the interventional device, or it may occur between the interventional device and the optical detector.

An interventional device is generally known in the art, and may include any one of an endoscope, a catheter, a biopsy needle.

An imaging system of the interventional device is commonly known in the art, and is understood to comprise any one of various embodiments of imaging systems, including a scanning fibre system.

Light is to be broadly construed as electromagnetic radiation comprising wavelength intervals including visible, ultraviolet (UV), near infrared (NIR), infra red (IR), x-ray. The term optical is to be understood as relating to light.

Spectral information is understood to be information related to one or more wavelengths of light. A continuous spectrum represents spectral information, but it is further understood, that a measured light intensity within a certain wavelength interval also represents spectral information.

In another embodiment according to the invention, the exit position and the entry position are spatially separated and spatially oriented so that the entry position is not intersected by ballistic photons emitted from the exit position, when the distal end of the interventional device is placed adjacent the associated sample. It is understood that the entry position is not intersected by ballistic photons emitted from the exit position, at least from a practical point of view. For all practical purposes, the number of ballistic photons hitting the entry position is non-zero but negligible.

Ballistic photons are construed as photons which move in straight lines without being scattered more than once, such as a photon used for imaging which is scattered once on the imaged object.

Diffusive photons are photons which experience multiple, scattering events, such as multiple random scattering events. The scattering events may be elastic, such as Rayleigh scattering, or inelastic, such as Raman scattering. Absorption of photons emitted at the exit position may take place at certain wavelengths giving rise to particular absorption bands being visible in the spectrum of the diffusive photons being collected at the entry position.

By arranging the entry and exit positions as described, a large majority of photons collected at the entry position will be diffusive photons which have traversed a relatively long and non-straight path between the exit and entry position. In total, when using a large number of photons, as will generally be the case, the information collected together with the photons collected at the entry position will be dependent on a second region, the second region being traversed by the diffusive photons emitted at the exit position, and the second region being larger than the imaged first region.

In yet another embodiment of the system, the photons emittable at the exit position and subsequently collectable at the entry position are diffusive photons. An advantage of collecting diffusive photons may be that in general they have traversed a larger region, compared to ballistic photons.

In yet another embodiment of the system, the photons exiting the second guide are non-focused. The photons may initially after exiting the second guide constitute paraxial or diverging rays, or they may otherwise be non-focused. It is understood that in the present context, the photons exiting the second guide are considered non-focused if they are not focused within a distance comparable to a spatial scale of the first region. A possible advantage of this is that the energy is divided over a broader area of the adjacent sample due to the defocusing, and as a result there is less risk of damaging the adjacent sample.

In another embodiment, the imaging system further comprises a movable element, such as a moving guide or a moving lens. When using, for instance, scanned optical illumination, the size and number of the photon detectors do not limit the resolution and number of pixels of the resulting image. Additional features may include enhancement of topographical features, stereoscopic viewing, and accurate measurement of feature sizes of a region of interest in a patient's body that facilitate providing diagnosis, monitoring, and/or therapy with the instrument.

In another embodiment according to the invention, the imaging system comprises a fibre bundle. Instead of using a scanning fibre as mentioned above, also a fibre bundle can be employed. A fibre bundle has for instance been used by the company Mauna Kea Technologies.

In yet another embodiment according to the invention, the interventional device further comprises a plurality of first guides and/or a plurality of second guides. A possible advantage of this may be that it enables spatial resolution of the average spectral information of the associated sample obtained via the spectrometer and the first and second guides. In other words, a plurality of regions may be examined, where these regions may be different regions which may be overlapping or non-overlapping. It is understood, that the invention encompasses an embodiment where the second region, which is larger than the first region, is constructed based on a plurality of such different regions.

In another embodiment according to the invention, the system further comprises any one of: a spectrometer for performing reflectance spectroscopy, a spectrometer for performing Raman spectroscopy, a spectrometer for performing fluorescence spectroscopy, an electrode, a microprobe, a thermometer and/or a force gauge. The microprobe may be advantageous for microdialysis, such as for measuring pH or glucose content. The force gauge may be advantageous for measuring elasticity.

In another embodiment according to the invention, the exit position and/or the entry position are situated on a side of the interventional device at the distal end of the interventional device. This may be advantageous, as it enables a distance between the position of emission of photons at the exit position and the position of collection of photons at the entry position which is larger than the diameter of the imaging optics, without having to make the end of the interventional device oblique. It is understood here, that the term 'diameter' may not be construed so as to limit the imaging fibre lens to circular configurations.

In yet another embodiment according to the invention, the system further comprises any one of: a light source for providing therapeutic light and/or an ultrasound unit. A possible advantage of providing a therapeutic light source is that it enables therapy using light. An advantage of providing an ultrasound unit may be that it enables ablation, such as radio frequency ablation (RFA) or imaging. There may further be feedback loops that connect a console which control of the tissue ablation process using algorithms that process signals from the first and second guide and the imaging system. The console may be a controlling system comprising a computer. In one embodiment, the first and/or second guide are connected via an optical switch to an optical source that can provide therapeutic light, for instance with photodynamic therapy (PDT). With a switch at one position, light could be delivered or received for sensing; at the other position, therapeutic light could be delivered. Therapeutic light could be delivered based on the information received from the combination of the first and second guide and the imaging system, and this delivery process could be implemented with a feedback loop. Such a feedback loop would connect a console in which algorithms that process signals from the spectrometer and the imaging system, and the optical switch and therapeutic light source.

In another embodiment according to the invention, the system further comprises a processor, the processor being arranged for
- receiving spectral information of photons collected at the entry position, and
- comparing the spectral information with stored information in the database, wherein the stored information comprises spectral information related to at least one type of sample.

An advantage of providing a processor as described may be that it enables a fast and/or automated comparison with database values.

In yet another embodiment of the invention, the processor is further arranged for
- receiving primary information from the imaging means,
- generating a primary image,
- receiving spectral information of photons collected at the entry position,
- generating a secondary image based on the spectral information of photons collected at the entry position, and
- combining the primary image and the secondary image into a tertiary image.

The tertiary image may comprise information related to the spectral information obtained from the spectrometer and the first and second guide, such as by having a certain colour or intensity in one or more border regions of the image, or by affecting a colour scale of the primary (microscope) image. In one embodiment, a grey scale of the primary (microscope) image is converted into a colour scale based on the spectral information from the spectrometer and the first and second guide.

In another embodiment according to the invention, the interventional device has
imaging optics capable of guiding light used for imaging a first region of an associated sample,
first guide for guiding photons from the second light source to an exit position on a distal end of the interventional device, and
a second guide for guiding photons from an entry position on the distal end of the interventional device and to the optical detector,
wherein the exit position and the entry position are spatially separated and spatially oriented so that the entry position is not intersected by relatively likely paths of ballistic photons emitted from the exit position, when the distal end of the interventional device is placed adjacent to the associated sample.

According to a second aspect of the invention, the invention further relates to a method for extending microscopy information, the method comprising the steps of,
- imaging a first region of an associated sample,
- performing a spectroscopic analysis of a second region of the associated sample, the spectroscopic analysis comprising the steps of
- guiding photons from the light source to the exit position, and
- guiding photons from an entry position and into the optical detector,
wherein the exit position and the entry position are spatially separated and spatially oriented so that so that an average spectral information of photons emitted from the exit position and collected at the entry position, is dependent on a second region of the associated sample, when the distal end of the interventional device is placed adjacent to the associated sample, the second region being larger than the first region.

According to a third aspect of the invention, the invention further relates to a computer program product being adapted to enable a computer system comprising at least one computer having a data storage means associated therewith to operate a processor arranged for
- receiving primary information from an imaging means,
- generating a primary image,
- receiving spectral information of photons collected at an entry position on an interventional device,
- generating a secondary image based on the spectral information of photons collected at the entry position on the interventional device, and
- combining the primary image and the secondary image into a tertiary image.

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The system according to the invention will now be described in more detail with regard to the accompanying figures. The figures show one way of implementing the present invention and is not to be construed as being limiting to other possible embodiments falling within the scope of the attached claim set.
Fig. 1 shows a side view of a part of an interventional device according to an embodiment of the invention,
Fig. 2 shows side views of a part of an interventional device according to other embodiments of the invention
Fig. 3 shows schematic end views of the distal part of an interventional device according to different embodiments according to the invention,
Fig. 4 shows schematic images generated according to an embodiment of the invention,
Fig. 5 shows a schematic of an embodiment of the system according to the invention, and
Fig. 6 shows a flow chart of a method for extending microscopy information with spectral information.

### DETAILED DESCRIPTION OF THE EMBODIMENTS

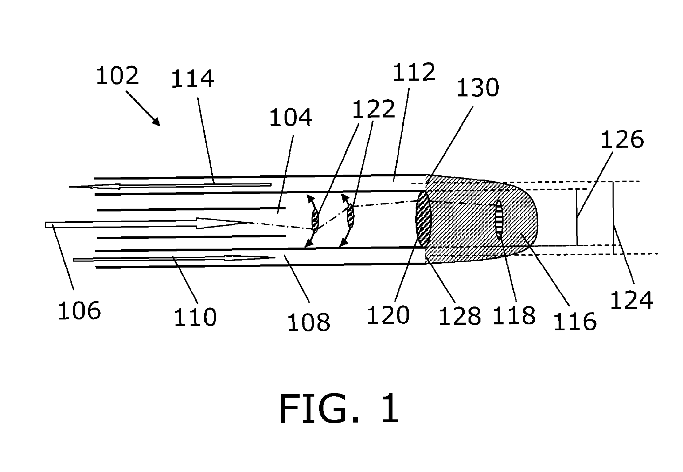

An embodiment of an interventional device 102 according to an embodiment of the invention is shown in Figure 1. The figure is showing part of an interventional device, which may be part of a scanning fibre imaging system extended with two fixed fibres. The interventional device comprises a first guide 108 and a second guide 112 for guiding light. The imaging system comprises an imaging guide 104 through which photons can travel along the guide, such as in the direction depicted with arrow 106. The imaging system further comprises a number of lenses, such as fixed lenses 120 and movable lenses such as 122. In some embodiments, a moving lens may be attached to the imaging guide 104 which may be moving. The imaging system is capable of and arranged for imaging a region of interest (ROI) herein also referred to as the first region 118. The first and the second guide and their respective end points at a distal end, exit position 128 and entry position 130, are spatially positioned in such a way that, when photons moving in a direction given by 110 through the first guide 108 are emitted at the exit position 128 and collected at the entry position 130 the probing volume also referred to as the second region 116 which they traverse, has a lateral dimension larger than the field of view of the scanning fibre system. A distance 126 between the end and entry points 128, 130 is larger than a width 124 of the optical elements of the imaging system.

In this particular embodiment, the scanning fibre system is based on a scanning the fibre with an electromagnetic motor. A more extensive description of this system can be found in patent applications WO2009/087522 and W02009/013663 which are hereby each incorporated by reference in its entirety.

Both the first and second guides 108, 112, which can be fixed, as well as the imaging guide 104 are connected to a console. The scanning fibre system may provide a microscope image of the associated sample, such as a tissue or a food sample, in front of the tip of the interventional device. The first and second guides 108, 112 may for instance provide information, such as a reflectance spectrum, from a second region 116 close to the tip of the interventional device 102. From this information parameters like blood content, blood oxygenation, water and fat content can be derived. This information on the global content of the second region 116 can be helpful additional information to understand and interpret the microscopic image which covers the first region 118. In the pathology department the pathologist knows in general at what type of tissue he/she is looking at beforehand and this helps in the interpretation. In case where we use an interventional device, such as a needle like device, and insert it in a sample, this context is lost. The information provided via the first and second guide 108, 112, being able to probe a large volume, such as the second region 116, gives already a clue of what type of tissue we are looking at. The microscopic image then gives the possibility to inspect the sample in a much deeper level, such as whether it is diseased or not.

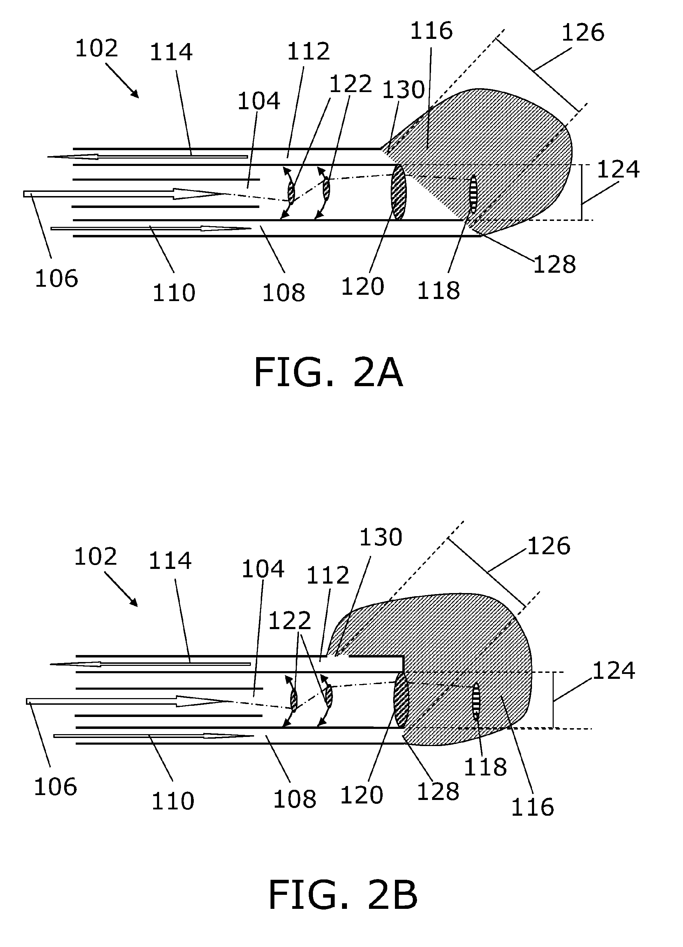

Figure 2A shows an embodiment which is similar to the embodiment depicted in Figure 1 except that and end section of the interventional device is oblique with respect to the lengthwise direction of the elongated interventional device.

Figure 2B shows another embodiment which is similar to the embodiment depicted in Figure 1 except that the entry point is positioned on the side of the distal end of the interventional device.

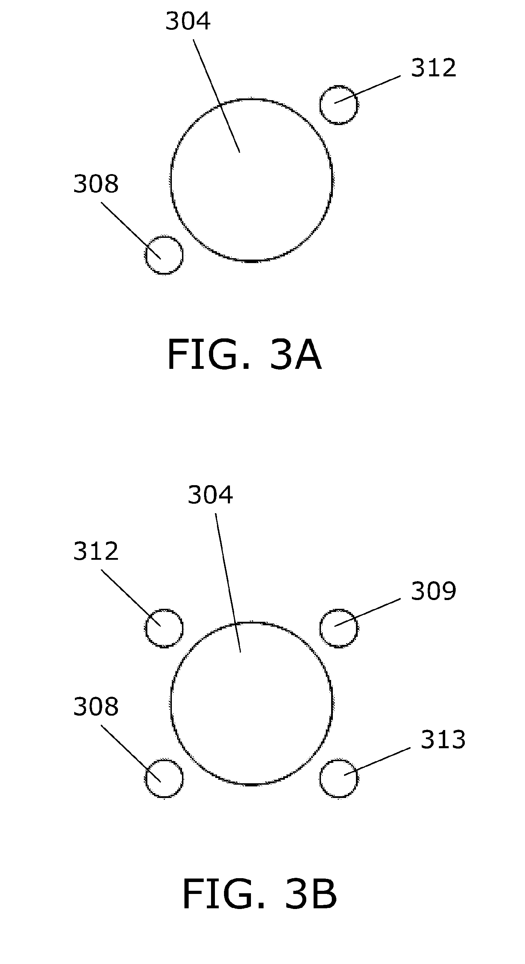

Figure 3A shows a very schematic end view of the distal end of interventional device. The imaging guide 304 is shown in the middle, with the first guide 308 on one side and the second guide 312 on the other side.

Figure 3B shows a very schematic end view of the distal end of interventional device according to another embodiment. This embodiment is similar to the embodiment depicted in Figure 3A, except that it contains more than one first guide and more than one second guide, which enables it to provide, spatial resolution of the associated sample in second regions probed by the first and second guides. In the figure, the imaging guide 304 is shown in the middle, with the first guide 308 on one side and the second guide 312 on the other side. Furthermore, another set of first and second guides, first guide 309 and second guide 313, are shown on each side of the imaging guide, and rotated 90 degrees around the imaging guide. First guides 308 and 309 can be attached to an optical source via an optical switch, and second guides 312 and 313 can be attached to a optical detector via a second optical switch. The region near, e.g., the top of the imaged region, which may be imaged using scanning, can be probed by connecting light guide 309 to the source and light guide 312 to the optical detector. Thus, by emitting photons from first guide 308 and collecting photons by second guide 312, a second region substantially located in the vicinity of these two guides. By emitting photons from first guide 308 and collecting photons by second guide 313, a second region substantially located in the vicinity of these two guides. Similarly, by emitting photons from first guide 309 and collecting photons by second guide 312 or second guide 313, second regions substantially located in the vicinity of first and second guide 309, 312 or 309 313 can be probed. Consequently, this embodiment provides enhanced spatial resolution.

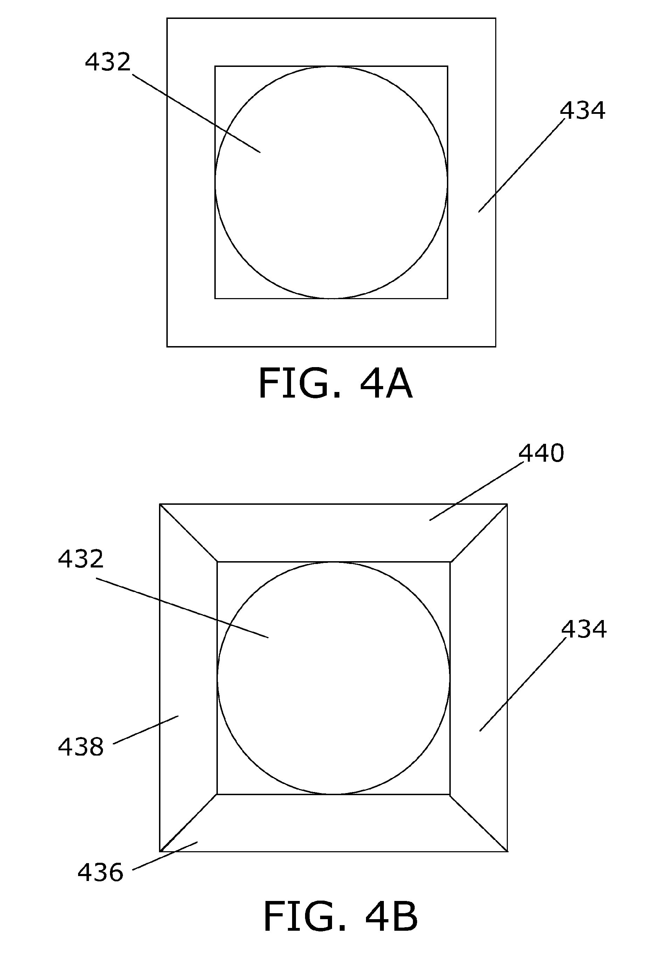

Figure 4A shows a schematic image generated according to an embodiment of the invention. The figure shows the microscope image 432 of the first region, depicted using the imaging system, and a border 434 which may change in appearance, such as change colour or intensity, dependent on the information collected from the second region.

Figure 4B shows a schematic image generated according to an embodiment of the invention. The figure shows the microscope image 432 of the first region, depicted using the imaging system, and a border split into sections 434, 436, 438, 440 which may each change in appearance, such as change colour or intensity, dependent on the information regarding the second region. The spatial resolution of the information collected from the second regions may be collected using an embodiment as depicted in Figure 3B. In one example, a fat-containing tissue region with an oxygen-rich region (e.g., an artery) near the top of the image may provide sections 434, 436, 438 to have appearances corresponding to `fat', while section 440 has another appearance corresponding to `oxygen-rich'.

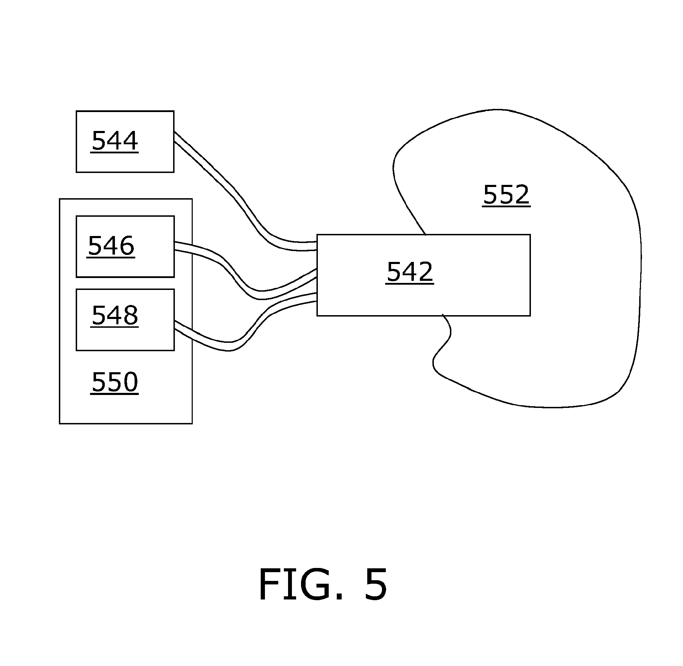

Figure 5 shows a schematic of an embodiment of the system according to the invention comprising a first light source 544 for imaging, a spectrometer 550 comprising a second light source 546, and an optical detector 548, and an interventional device 542, where the interventional device 542 has imaging optics capable of guiding light from the first light source so to perform imaging of a first region of an associated sample 552.

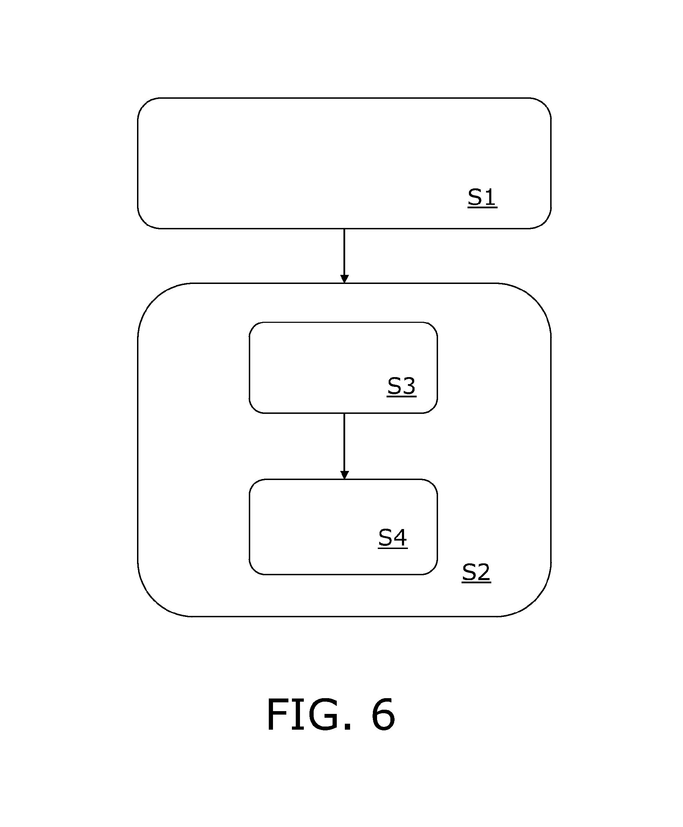

Figure. 6 is a flow chart of a method for extending microscopy information with spectral information, the method comprising the steps of,
- imaging (S1) a first region of an associated sample,
- performing (S2) a spectroscopic analysis of a second region of the associated sample, the spectroscopic analysis comprising the steps of
- guiding (S3) photons from the light source to the exit position, and
- guiding (S4) photons from an entry position and into the optical detector,
wherein the exit position and the entry position are spatially separated and spatially oriented so that so that an average spectral information of photons emitted from the exit position and collected at the entry position, is dependent on a second region of the associated sample, when the distal end of the interventional device is placed adjacent to the associated sample, the second region being larger than the first region.

To sum up, the present invention relates to a system for extending microscopy information, where the microscopy information is image information from a first region 118 of an associated sample, the first region being imaged with an imaging system. The extension of the microscopy information originates from probing a larger second region 116 by photons which are emitted at an exit position 128 and collected at en entry position 130. The exit position and the entry position are spatially separated so that so that average spectral information of photons emitted from the exit position and collected at the entry position, is dependent on the second region 116 of the associated sample, the second region 116 being larger than the first region 118.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A system for obtaining extended microscopy information, comprising
- a first light source (544) for imaging,
- a spectrometer (550) comprising
- a second light source (546), and
- an optical detector (548), and
- an interventional device (542), where the interventional device (542) has
- imaging optics capable of guiding light from the first light source (544) so to perform imaging of a first region (118) of an associated sample (552),
- a first guide (108) for guiding photons from the second light source (546) to an exit position (128) on a distal end of the interventional device, the photons being emittable from the exit position (128), and
- a second guide (112) for guiding photons from an entry position (130) on the distal end of the interventional device and to the optical detector (548),
wherein the exit position (128) and the entry position (130) are spatially separated and spatially oriented so that, upon positioning the distal end of the interventional device adjacent to the associated sample, an average spectral information is obtainable from photons collectable at the entry position, the average spectral information comprises information about a second region (116) of the associated sample, and
wherein the exit position and the entry position are arranged so that the second region (116) is larger than the first region (118).

2. A system according to claim 1, wherein the exit position and the entry position are spatially separated and spatially oriented so that the entry position is not intersected by ballistic photons emitted from the exit position, when the distal end of the interventional device is placed adjacent the associated sample.

3. A system according to claim 1, wherein the photons emittable at the exit position and subsequently collectable at the entry position are diffusive photons.

4. A system according to claim 1, wherein the photons exiting the second guide are non-focused.

5. A system according to claim 1, wherein the imaging system comprises a movable element.

6. A system according to claim 1, wherein the imaging system comprises a fibre bundle.

7. A system according to claim 1, wherein the interventional device further comprises a plurality of first guides and/or a plurality of second guides.

8. A system according to claim 1, further comprising any one of: a spectrometer for performing reflectance spectroscopy, a spectrometer for performing Raman spectroscopy, a spectrometer for performing fluorescence spectroscopy, an electrode, a microprobe, a thermometer and/or a force gauge.

9. A system according to claim 1, wherein the exit position and/or the entry position are situated on a side of the interventional device at the distal end of the interventional device.

10. A system according to claim 1, wherein the system further comprises any one of: a light source for providing therapeutic light and/or an ultrasound unit.

11. A system according to claim 1, further comprising a processor, the processor is being arranged for
- receiving spectral information of photons collected at the entry position, and
- comparing the spectral information with stored information in the database,
wherein the stored information comprises spectral information related to at least one type of sample.

12. A system according to claim 11, wherein the processor is further arranged for
- receiving primary information from the imaging means,
- generating a primary image,
- receiving spectral information of photons collected at the entry position,
- generating a secondary image based on the spectral information of photons collected at the entry position, and
- combining the primary image and the secondary image into a tertiary image.

13. An interventional device, where the interventional device has imaging optics capable of guiding light used for imaging a first region of an associated sample,
first guide for guiding photons from the second light source to an exit position on a distal end of the interventional device, and
a second guide for guiding photons from an entry position on the distal end of the interventional device and to the optical detector,
wherein the exit position and the entry position are spatially separated and spatially oriented so that the entry position is not intersected by relatively likely paths of ballistic photons emitted from the exit position, when the distal end of the interventional device is placed adjacent to the associated sample.

14. A method for extending microscopy information, the method comprising the steps of,
- imaging (S1) a first region of an associated sample,
- performing (S2) a spectroscopic analysis of a second region of the associated sample, the spectroscopic analysis comprising the steps of
- guiding (S3) photons from the light source to the exit position, and
- guiding (S4) photons from an entry position and into the optical detector,
wherein the exit position and the entry position are spatially separated and spatially oriented so that so that an average spectral information of photons emitted from the exit position and collected at the entry position, is dependent on a second region of the associated sample, when the distal end of the interventional device is placed adjacent to the associated sample, the second region being larger than the first region.

15. A computer program product being adapted to enable a computer system comprising at least one computer having a data storage means associated therewith to operate a processor arranged for
- receiving primary information from an imaging means,
- generating a primary image,
- receiving spectral information of photons collected at an entry position on an interventional device,
- generating a secondary image based on the spectral information of photons collected at the entry position on the interventional device, and
- combining the primary image and the secondary image into a tertiary image.
